(19) Europäisches Patentamt / European Patent Office / Office européen des brevets

(11) **EP 3 463 032 B1**

(12) **EUROPEAN PATENT SPECIFICATION**

(45) Date of publication and mention of the grant of the patent:
**23.12.2020   Bulletin 2020/52**

(21) Application number: **17726932.1**

(22) Date of filing: **30.05.2017**

(51) Int Cl.:
*A61B 1/00* (2006.01)          *A61B 8/00* (2006.01)
*A61B 90/00* (2016.01)          *A61B 34/10* (2016.01)

(86) International application number:
**PCT/EP2017/063031**

(87) International publication number:
**WO 2017/207565 (07.12.2017 Gazette 2017/49)**

(54) **IMAGE-BASED FUSION OF ENDOSCOPIC IMAGE AND ULTRASOUND IMAGES**

BILDBASIERTE FUSION VON ENDOSKOPISCHEN BILDERN UND ULTRASCHALLBILDERN

FUSION À BASE D'IMAGE ENDOSCOPIQUE ET D'IMAGES ÉCHOGRAPHIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **31.05.2016   US 201662343339 P**

(43) Date of publication of application:
**10.04.2019   Bulletin 2019/15**

(73) Proprietor: **Koninklijke Philips N.V.**
**5656 AG Eindhoven (NL)**

(72) Inventors:
• **POPOVIC, Aleksandra**
  **5656 AE Eindhoven (NL)**

• **THIENPHRAPA, Paul**
  **5656 AE Eindhoven (NL)**
• **TOPOREK, Grzegorz, Andrzej**
  **5656 AE Eindhoven (NL)**

(74) Representative: **Philips Intellectual Property & Standards**
**High Tech Campus 5**
**5656 AE Eindhoven (NL)**

(56) References cited:
**WO-A1-2015/091226     WO-A1-2016/009339**
**US-A1- 2009 036 902     US-A1- 2014 303 491**

**Description**

FIELD OF THE INVENTION

The present disclosure generally relates to minimally invasive procedures involving an endoscopic view of a laparoscope imaging of an anatomical region (e.g., cardiac surgery, laparoscopic surgery, natural orifice transluminal surgery, single incision laparoscopic surgery, pulmonary/bronchoscopy surgery and diagnostic interventions). The present disclosure more particularly relates to an image-based fusion of an endoscopic image and a ultrasound image of the anatomical region.

BACKGROUND OF THE INVENTION

Endoscopes and laparoscopes are thin, elongated camera assemblies that allow clinicians to view the internal anatomy of a patient without the need to surgically expose the anatomy for a direct view. Endoscopes can fit through narrow natural orifices or small incisions in the skin, thus resulting in reduced trauma to the patient as compared to open surgery.

Laparoscopic ultrasound (LUS) refers to any ultrasound imaging device that is introduced through a port (or a natural orifice) into the internal anatomy of a patient and used to image internal organs. Two examples of LUS probes includes the Philips L10-41ap probe and the Philips C9-3io probe.

More particularly, LUS is used to assess internal organs and identify surgery targets (e.g., tumors) and identify sensitive structures inside those organs (e.g. blood vessels). In clinical practice today, LUS probes are introduced into the body through one of the ports for surgical instruments. The organ(s) is(are) scanned to assess location of various structures and targets. After the assessment, the LUS probe is removed from the port and instruments are introduced into the body through the port(s) to complete the procedure. If subsequently needed, the LUS probe can be reintroduced into the body at a different time.

Tracking-based fusion as practiced in the art relies primarily on external tracking devices (e.g., optical tracking and/or electromagnetic tracking) for both tracking of a laparoscopic ultrasound probe and ultrasound-endoscopic image fusion, thus making this practice very complex and cumbersome to utilize during minimally invasive procedures.

Additionally, there are several main problems with the surgical use of LUS.

First, an operator knows the tracking-based correspondence between the endoscopic image to the ultrasound image(s) only at the time of an operator scanning of the anatomical region by the LUS probe as the endoscope and the LUS probe are being tracked. Once the LUS scan is complete and the LUS probe is removed from the body, the relationship between the endoscopic image to the ultrasound image(s) is lost.

Second, handling of the LUS probe is complex due to elongated nature of the LUS probe and the fulcrum effect allowing limited motion of the LUS probe around a single entry point.

Third, tracking of the LUS probe can solve some of the problems, however it introduces a need for more equipment in the operating theatre impairing the workflow. In addition, if optical tracking is used, it is difficult to accurately infer the position of the LUS probe using the external marker.

WO2015091226 discloses a system for providing laparoscopic vision to a surgeon for treating a patient body during invasive surgery, which has a processing device that receives acquired image, laparoscopic image and signal from sensor, and display for displaying combined view.

SUMMARY OF THE INVENTION

To improve upon the advantages and benefits of laparoscopic imaging of an anatomical region during a minimally invasive procedure, the present disclosure provides systems, workstations, controllers and methods to integrate an endoscopic image of an anatomical region with laparoscopic ultrasound (LUS) image(s) of the anatomical region and to display the integrated information during an operator scanning of the anatomical region or at any other time during the procedure. The systems, workstations, controllers and methods of the present disclosure implement imaged-based fusion of endoscopic image and ultrasound image(s), which eliminates a need for additional tracking devices (e.g., optical tracking and/or electromagnetic tracking). The imaged-based fusion of the present disclosure will enable more intuitive use of LUS probes and will enable better targeting during the procedure (e.g., cardiac surgery, laparoscopic surgery, natural orifice transluminal surgery, single incision laparoscopic surgery, pulmonary/bronchoscopy surgery and diagnostic interventions).

One form of inventions of the present disclosure is an image fusion workstation for an image-based fusion of an endoscopic image of an anatomical region generated by an endoscope (e.g., an endoscopic view of an anatomical organ within the anatomical region) and an ultrasound image of the anatomical region generated by a laparoscopic ultrasound probe (e.g., an ultrasound view of the anatomical organ within the anatomical region). The image fusion workstation employs an image fusion controller controlling the fusion between the endoscopic image and the ultrasound image based on an image transformation between an endoscopic image space of the endoscope and an ultrasound image space of the laparoscopic ultrasound probe derived from a detection by the image fusion controller of the laparoscopic ultrasound probe within a field-of-view of the endoscope. The workstation further employs a display controller controlling a display of the fusion by the image fusion controller of the endoscopic image and the ultrasound image (e.g., a registered overlay of the ultrasound image on the endoscopic image, or a registered window display of the ultrasound sound image relative to a display of the endoscopic image).

A second form of the inventions of the present disclosure is the image fusion controller employing a probe detector, an image transformer and an image integrator. The probe detector controls a detection of the laparoscopic ultrasound probe within a field-of-view of the endoscope. The image transformer controls a computation of an image transformation between an endoscopic image space of the endoscope and an ultrasound image space of the laparoscopic ultrasound probe derived from the detection by the probe detector of the laparoscopic ultrasound probe within the field-of-view of the endoscope. The image integrator controls the fusion of the endoscopic image and the ultrasound image based on the image transformation computed by the image transformer.

A third form of the inventions of the present disclosure is a method for an image-based fusion of an endoscopic image of an anatomical region generated by an endoscope (e.g., an endoscopic view of an anatomical organ within the anatomical region) and an ultrasound image of the anatomical region generated by a laparoscopic ultrasound probe (e.g., an ultrasound view of the anatomical organ within the anatomical region). The method involves an image fusion workstation detecting the laparoscopic ultrasound probe within a field-of-view of the endoscope, and computing an image transformation between an endoscopic image space of the endoscope and an ultrasound image space of the laparoscopic ultrasound probe, wherein the image fusion workstation derives the image transformation from the detection of the laparoscopic ultrasound probe within the field-of-view of the endoscope. The method further involves the image fusion workstation fusing the endoscopic image and the ultrasound image based on the image transformation.

All of the inventions of the present disclosure may additionally incorporate a fusion of the endoscopic/ultrasound images to an anatomical model in the form of

(1) an volume image of the anatomical region generated by an imaging modality (e.g., a computed tomography (CT or XperCT), magnetic resonance image (MRI), positron emission tomography (PET), etc. view of an anatomical organ within the anatomical region), or
(2) an anatomical atlas of an anatomical organ within the anatomical region.

From an anatomical model registration, the display of the fusion of endoscopic/ultrasound images may include:

(1) the anatomical model being displayed with the endoscopic image (e.g., a registered overlay of reference planes of the anatomical model on the endoscopic image),
(2) the anatomical model being displayed with the ultrasound image (e.g., a registered overlay of the ultrasound image on the anatomical model), and/or
(3) the anatomical model being displayed with the fusion of the endoscopic/ultrasound image (e.g., the registered overlay of both the ultrasound image and reference planes of the anatomical model on the endoscopic image).

For purposes of the present disclosure, terms of the art including, but not limited to, "fusion", "anatomical region", "anatomical organ", "endoscope", "endoscopic image", "field-of-view", "laparoscopic ultrasound probe", "ultrasound image", "image transformation", "registration", "image space", "display", "volume image", "imaging modality" and "anatomical atlas" are to be interpreted as understood in the art of the present disclosure and as exemplary described herein.

More particularly for purposes of the present disclosure, the term "endoscope" broadly encompasses any device structurally configured with ability to image from inside a body as understood in the art of the present disclosure and as exemplary described herein.

Examples of an endoscope include, but are not limited to, any type of scope, flexible or rigid (e.g., endoscope, arthroscope, bronchoscope, choledochoscope, colonoscope, cystoscope, duodenoscope, gastroscope, hysteroscope, laparoscope, laryngoscope, neuroscope, otoscope, push enteroscope, rhinolaryngoscope, sigmoidoscope, sinuscope, thorascope, colposcope, thoracoscope, sygmoidscope, neuroendoscope, etc.) and any device similar to a scope that is equipped with an image system). The imaging is local, and surface images may be obtained optically with fiber optics, lenses, and miniaturized (e.g. CCD based) imaging systems (e.g., laparoscopic ultrasound).

For purposes of the present disclosure, the term "controller" broadly encompasses all structural configurations of an application specific main board or an application specific integrated circuit housed within or linked to a workstation for controlling an application of various inventive principles of the present disclosure as subsequently described herein. The structural configuration of the controller may include, but is not limited to, processor(s), computer-usable/computer readable storage medium(s), an operating system, application module(s), peripheral device controller(s), slot(s) and port(s).

Any descriptive labeling of a controller herein (e.g., a "endoscope controller", "LUS probe controller", "image fusion controller" and "display controller") serves to identify a controller as described and claimed herein without specifying or implying any additional limitation to the term "controller".

Examples of the workstation include, but are not limited to, an assembly of one or more computing devices, one or more input devices (e.g., a keyboard, joysticks and mouse) and one or more display/monitors (e.g., a client computer, a desktop, a laptop and a tablet).

For purposes of the present disclosure, the term "application module" broadly encompasses a component of the controller consisting of an electronic circuit and/or an executable program (e.g., executable software and/or firmware stored on non-transitory computer readable medium(s)) for executing a specific application. Any descriptive labeling of an application module herein (e.g., a "probe detector", "image transformer" and "image integrator") serves to identify a particular application module as described and claimed herein without specifying or implying any additional limitation to the term "application module".

The foregoing forms and other forms of the inventions of the present disclosure as well as various features and advantages of the inventions of the present disclosure will become further apparent from the following detailed description of various embodiments of the inventions of the present disclosure read in conjunction with the accompanying drawings. The detailed description and drawings are merely illustrative of the inventions of the present disclosure rather than limiting, the scope of the present inventions of the present disclosure being defined by the appended claims and equivalents thereof.

BRIEF DESCRIPTION OF THE DRAWINGS

FIG. 1 illustrates an exemplary embodiment of a minimally invasive procedure and controller suite in accordance with the inventive principles of the present disclosure.

FIG. 2 illustrates an exemplary embodiment of a minimally invasive control system in accordance with the inventive principles of the present disclosure.

FIG. 3 illustrates a flowchart representative of an exemplary embodiment of an image-based fusion method in accordance with the inventive principles of the present disclosure.

FIGS. 4A and 4B illustrate an image-based fusion of an endoscopic image and an ultrasound image in accordance with the inventive principles of the present disclosure.

FIG. 5 illustrates a registration of an endoscopic image and a pre-operative volume image as well known in the art.

FIG. 6 illustrates an exemplary embodiment of a registration of an image-based fusion of an endoscopic image and an ultrasound image to an anatomical atlas in accordance with the inventive principles of the present disclosure.

FIGS. 7-16 illustrate exemplary embodiments of a display of an image-based fusion of an endoscopic image and an ultrasound image in accordance with the inventive principles of the present disclosure.

FIGS. 17A and 17B illustrate exemplary embodiments of an image-based fusion workstation in accordance with the inventive principles of the present disclosure.

DETAILED DESCRIPTION OF THE PREFERRED EMBODIMENTS

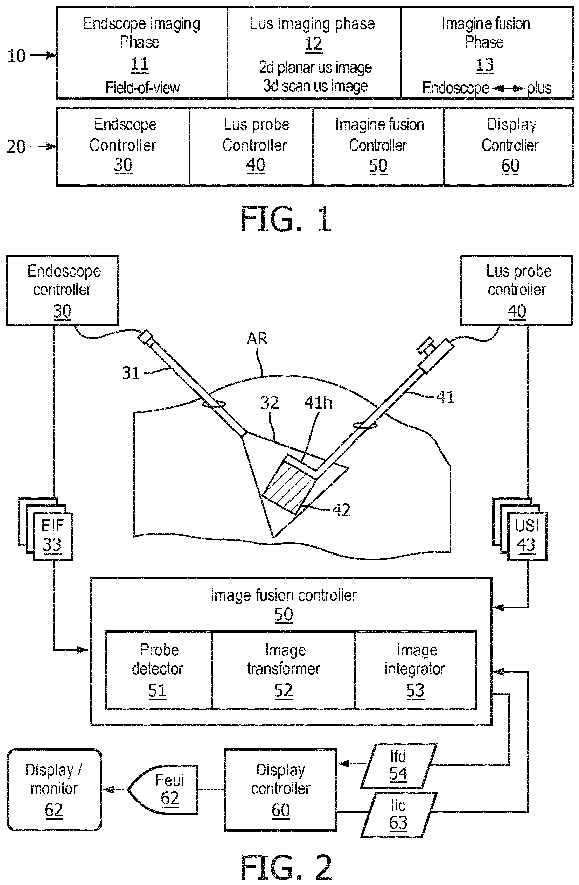

To facilitate an understanding of the present disclosure, the following description of FIGS. 1 and 2, teaches basic inventive principles of an image-based fusion 10 of an endoscopic image and an ultrasound image of an anatomical region for a minimally invasive procedure accomplished by a controller network 20. From this description, those having ordinary skill in the art will appreciate how to apply the inventive principles of the present disclosure to incorporate an image-based fusion of the present disclosure into various types of minimally invasive procedures.

Referring to FIG. 1, an image-based fusion 10 includes an endoscope imaging phase 11, a laparoscopic ultrasound (LUS) imaging phase 12, and an image fusion phase 13 incorporated within a minimally invasive procedure involving an anatomical region (e.g., an abdominal region).

Generally, endoscope imaging phase 11 involves an introduction of an endoscope through a port of a patient into the anatomical region as well known in the art of the present disclosure whereby the endoscope is operated to generate an endoscopic image of an anatomical region (e.g., an endoscopic view of an organ within the anatomical region) as will be further described herein in connection with FIG. 2.

Prior to, concurrent with or subsequent to endoscope imaging phase 11, LUS imaging phase 12 generally involves an introduction of a LUS probe through another port of a patient into the anatomical region as well known in the art of the present disclosure whereby the LUS probe is operated to generate a 2D planar ultrasound image of the anatomical region (e.g., an 2D ultrasound view of an organ within the anatomical region) or a 3D scan ultrasound image of the anatomical region consisting of a spatial sequence of 2D planar ultrasound images (e.g., a 3D ultrasound view of an organ within the anatomical region) as will be further described herein in connection with FIG. 2.

From the generated endoscopic/ultrasound images, image fusion phase 13 involves an image-based fusion in accordance with the inventive principles of the present disclosure premised on a detection of the LUS probe within a field-of-view of the endoscope as will be further described herein in connection with FIG. 2.

Upon an initial fusion of the endoscopic/ultrasound images, the LUS probe may be removed from the anatomical region as well known in the art of the present disclosure whereby surgical/diagnostic instrument(s) may be introduced into the anatomical region and a display of the image-based fused endoscopic/ultrasound images supports a navigation and an operation of the surgical/diagnostic instrument(s) within the anatomical region. Phases 11-13 may thereafter be individually/collectively repeated as necessary to complete the minimally invasive procedure.

To implement image-based fusion 10, controller network 20 employs an endoscope controller 30, a LUS probe controller 40, an image fusion controller 50 and a display controller 60.

Generally, endoscope controller 30 is structurally configured as well known in the art of the present disclosure for controlling an operation of an endoscope in generating an endoscopic image, LUS probe controller 40 is structurally configured as well known in the art for controlling an operation of a LUS probe in generating ultrasound image(s), and display controller 60 is structurally configured as well known in the art for controlling an operation of a display/monitor.

Image fusion controller 50 is structurally configured in accordance with the inventive principles of the present disclosure for controlling an image-based fusion of the endoscopic image and the ultrasound image premised on a detection of the LUS probe within a field-of-view of the endoscope as will be further described herein in connection with FIG. 2.

Display controller 60 may be further structurally configured in accordance with the inventive principles of the present disclosure to display a graphical user interface for providing user interactive commands to image fusion controller 50 to thereby manipulate the display of the image-based fusion of the endoscopic image and the ultrasound image.

In practice, controller network 20 may be incorporated within a single workstation or distributed in any manner among multiple workstations.

FIG. 2 illustrates an exemplary implementation of image-based fusion 10 by controller network 20 for a minimally invasive procedure involving an abdominal region AR.

Referring to FIGS. 1 and 2, endoscope controller 30 controls a generation of an endoscopic image by an endoscope 31 as well known in the art of the present disclosure. More particularly, endoscope 31 has a field-of-view 32 that may be fixed or adjustable by endoscope controller 30, and endoscope controller 30 employs a video capture device for converting an endoscopic video signal from endoscope 31 into a computer readable temporal frame sequence illustrative of the endoscopic image. In practice, the video capture device may employ a frame grabber of any type for capturing individual digital still endoscopic image frames ("EIF") 33 from the endoscopic video signal.

LUS probe controller 40 controls a generation of one or more ultrasound images by a LUS probe 41 as well known in the art of the present disclosure. More particularly, LUS probe 41 has a transducer head 41h for transmitting a short pulse of ultrasound longitudinally within an imaging plane 42 and for receiving reflected sound ("ultrasound echo") illustrative of an ultrasound image. LUS probe 41 may be stationary during imaging resulting in single 2D planar ultrasound image 43 or LUS probe 41 may be pivoted relative to the port resulting in a 3D scan ultrasound image consisting of a spatial sequence of 2D planar ultrasound images 43.

Image fusion controller 50 employs a probe detector 51, an image transformer 52 and an image integrator 53.

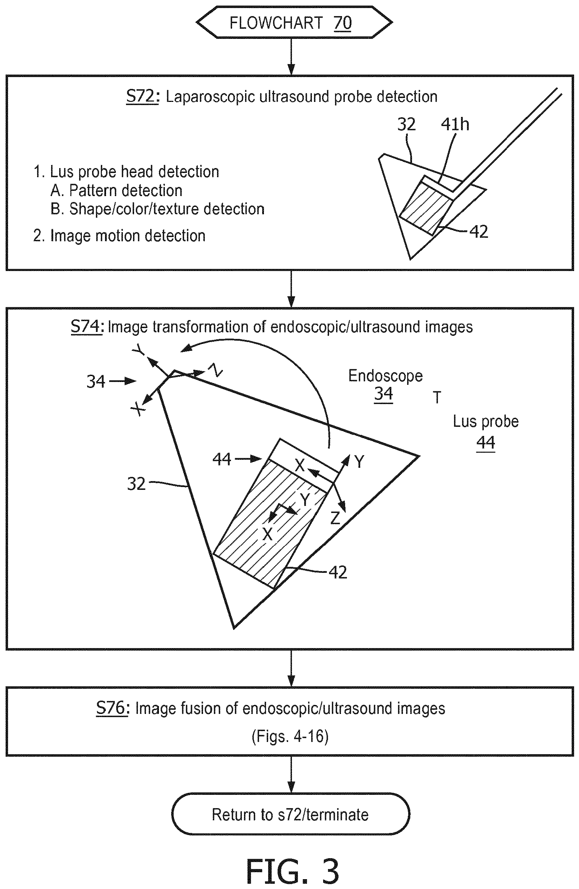

Probe detector 51 controls a processing of endoscopic image frames 33 to detect laparoscopic ultrasound probe 41 within field-of-view 32 of endoscope 31 as will be further described herein in connection with FIG. 3.

Image transformer 52 controls a processing of endoscopic image frames 33 and ultrasound image(s) 43 to compute an image transformation $^{ENDOSCOPE}T_{LUS\ PROBE}$ between an endoscopic image space of the endoscope 31 (e.g., field-of-view 31 or a portion thereof) and an ultrasound image space of LUS probe 41 (e.g., imaging plane 42 or a portion thereof) derived from the detection by probe detector 51 of LUS probe 41 within field-of-view 32 of endoscope 31 as will be further described herein in connection with FIG. 3.

Image integrator 53 controls a processing of endoscopic image frames 33 and ultrasound image(s) 43 to fuse the endoscopic image and the ultrasound image(s) based on the image transformation computed by image transformer 52 as will be further described herein in connection with FIG. 3.

Display controller 60 controls an operation of a display/monitor 62 as well known in the art of the present disclosure for illustrating the endoscopic image and the ultrasound image(S) 43, individually or as fused by image integrator 53. As to the fused endoscopic/ultrasound images, image integrator 52 provides image fusion data ("IFD") 54 to display controller 60 whereby display controller 60 generates a fused endoscopic image/ultrasound image ("FEUI") 61 for display by display/monitor 62. If interactive, display controller 60 provides interactive image commands "IIC") 63 via a graphical user interface (not shown) to image integrator 53 for manipulating the display of the fused endoscopic image/ultrasound image 61 by display/monitor 62

To facilitate a further understanding of the present disclosure, the following description of FIG. 3 teaches basic inventive principles of image-based fusion methods of the present disclosure in the context of the control network of FIG. 2. From this description, those having ordinary skill in the art will appreciate how to apply the inventive principles of the image-based fusion methods to numerous additional control network embodiments of the present disclosure.

FIG. 3 illustrates a flowchart 70 representative of one image-based fusion method of the present disclosure.

Referring to FIG. 3, a stage S72 of flowchart 70 encompasses probe detector 51 (FIG. 2) detecting LUS probe 41 within a field-of-view 32 of endoscope 31.

In one embodiment of stage S72, probe detector 41 automatically detects the distal transducer head 41h of LUS probe 41 within a field-of-view 32 of endoscope 31 by executing techniques known in the art based on a detection of a pattern of transducer head 41h, a specific shape/color/texture of transducer head 41 or a supplemental/additional pattern on transducer head 41h.

In a second embodiment of stage S72, probe detector 41 automatically detects the distal transducer head 41h of LUS probe 41 within a field-of-view 32 of endoscope 31 by executing detection techniques well known in the art of the present disclosure based on a detection of motion within the endoscopic image and/or ultrasound image(s) (e.g., optical flow, background extraction, etc.).

A stage S74 of flowchart 70 encompasses image transformer 52 (FIG. 2) attaching a reference frame 44 to transducer head 41h to thereby execute a 2D-3D registration as well known in the art (e.g., a RANSAC registration) for computing the image transformation $^{ENDOSCOPE}T_{LUS\ PROBE}$ between an endoscopic image space 34 of endoscope 31 and an ultrasound image space 42 of LUS probe 41.

A stage S76 of flowchart 70 encompasses image integrator 53 (FIG. 2) fusing the endoscopic image and the ultrasound image(s) to thereby facilitate a concurrent display of the fused endoscopic/ultrasound images including, but not limited to, a registered overlay of the ultrasound image on the endoscopic image, or a registered window display of the ultrasound sound image relative to a display of the endoscopic image.

In one embodiment of stage S76, an image blending technique as well known in the art of the present disclosure may be executed by image integrator 53 whereby this fusion process may include a perspective transformation of a 2D planar ultrasound image or a 3D transformation of a 3D scan ultrasound image.

In practice, stages S72-S76 may be performed for each imaging position of the LUS probe 41 within the anatomical region, or imaging position(s) of LUS probe 41 at specific time markers (e.g. 5 seconds) or for imaging position(s) of the LUS probe 41 selected by the operator.

Transformation $^{Endoscope}T_{LUS}$ may be saved by image fusion controller 50 alongside ultrasound images for each imaging position of LUS probe 41 during scanning or for imaging positions of LUS probe 41 selected by the operator.

Once of LUS probe 41 is removed from the anatomical region (e.g., abdominal region AR shown in FIG. 1), the operator may initiate display of fused endoscopic/ultrasound image via a combination of saved transformation $^{Endoscope}T_{LUS}$, the endoscopic image and the ultrasound image(s) by utilizing any interaction method including, but not limited to, a mouse (or other input device) click on the screen, saved positions (e.g. A, B,C) or by assigned names.

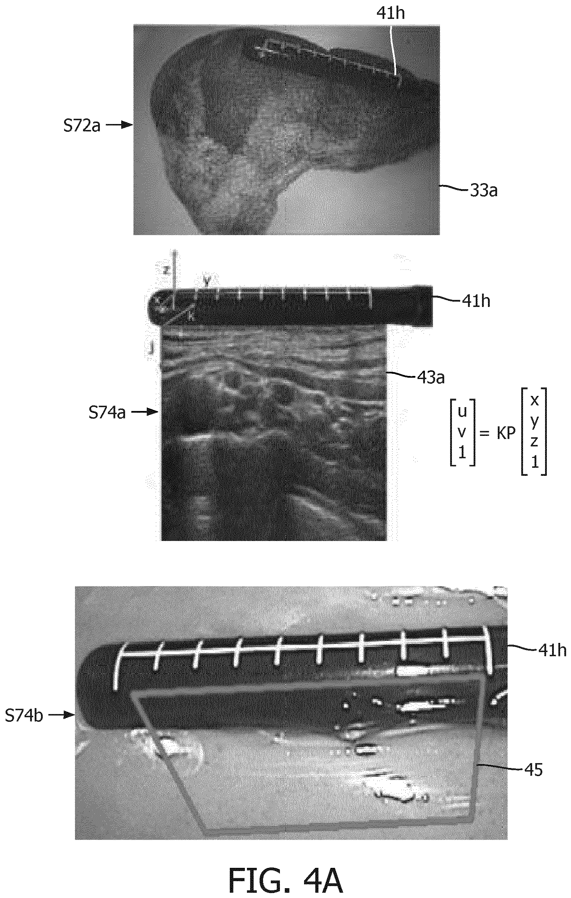

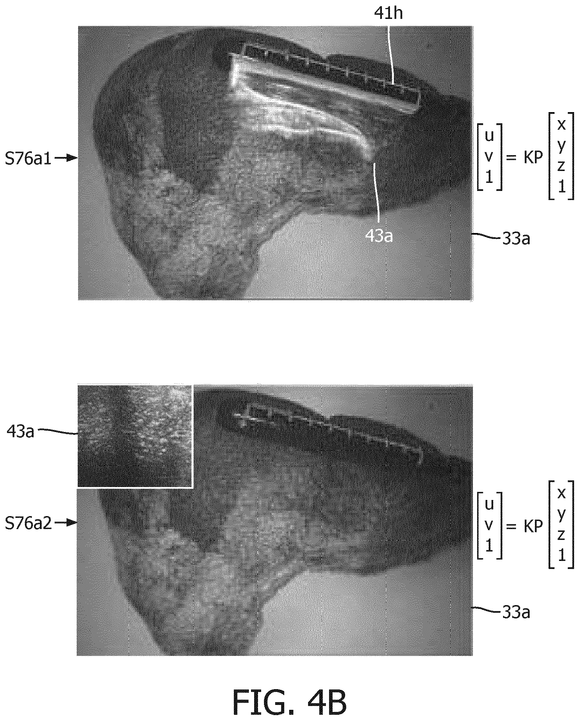

FIGS. 4A and 4B illustrate an exemplary execution of flowchart 70 involving:

1. Stage S72a: an automatic pattern detection of transducer head 41h of LUS probe 41 within field-of-view of an endoscopic image 33a showing an endoscopic view of an anatomical organ in the form of a liver;

2. Stage 74a: a mapping between the endoscopic image space (pixels) of endoscope 31 and the ultrasound image space of LUS probe 41 generating ultrasound image 43a providing an ultrasound view of a segment of the liver;

3. Stage 74b: a delineation of an outline 45 of the ultrasound image 43a;

4A. Stage 76a1: a utilization of outline 45 to display a fusion of endoscopic/ultrasound images 33a/43a in the form of a perspective overlay of ultrasound image 43a on endoscopic image 33a; and

4B. Stage 76a2: a utilization of outline 45 to display a fusion of endoscopic/ultrasound images 33a/43a in the form of a window display of ultrasound image 43a in an upper left hand corner of endoscopic image 33a.

Referring back to FIG. 3, stage S76 of flowchart 70 may further encompass image integration 53 executing an additional fusion of the endoscopic/ultrasound images to an anatomical model including, but not limited to, a volume image of the anatomical region and an anatomical atlas of an anatomical organ within the anatomical region.

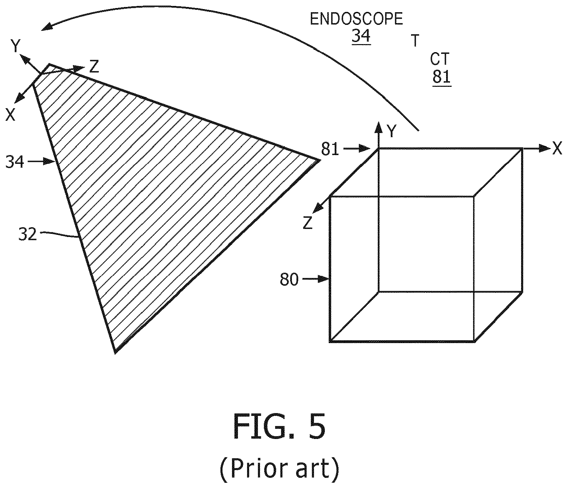

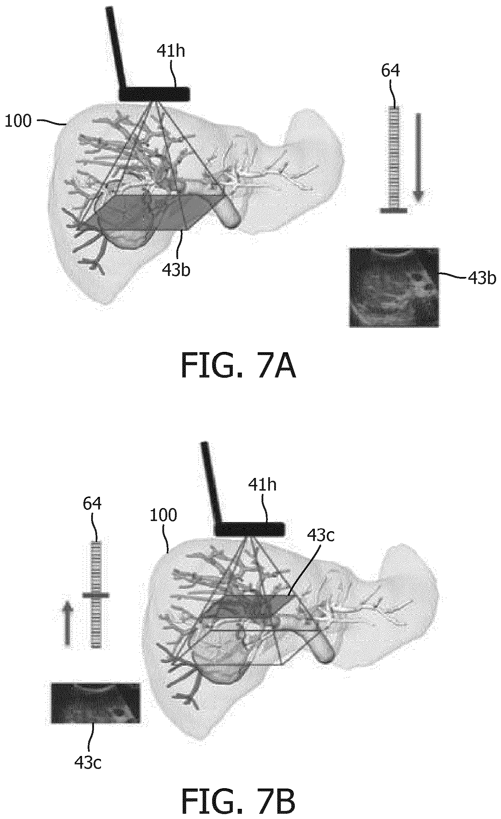

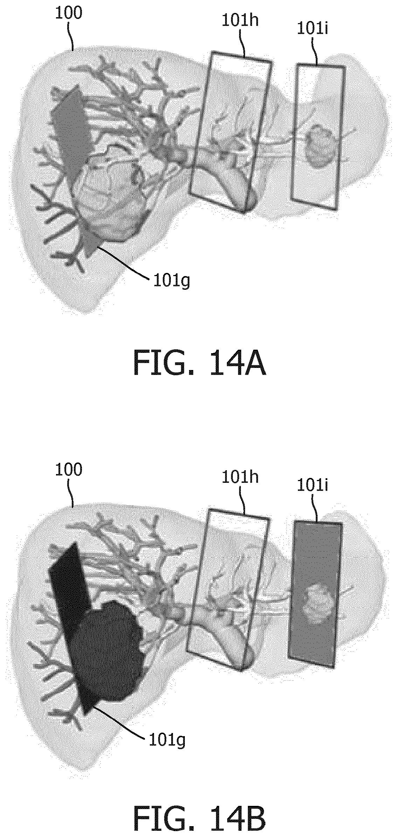

Volume Image Registration. The endoscope view may be registered to a preoperative 3D image (e.g., CT, XperCT, MRI, PET etc.) using methods well known in art of the present disclosure (e.g., U.S. Patent No. 9095252 B2). For example, FIG. 5 illustrates a registration $^{ENDOSCOPE}T_{CT}$ between a volumetric space 81 of a pre-operative CT image 80 and endoscopic image space 34 of endoscope 31 whereby 3D image and structures from pre-operative CT image 80 may be concurrently displayed based on the fusion of endoscope/ultrasound images as will be further explained in connection with FIGS. 7-16.

The $^{ENDOSCOPE}T_{CT}$ registration can be refined using an image-based registration method well known in art of the present disclosure (e.g. mutual information) acquired at a plane of interest. From $^{ENDOSCOPE}T_{CT}$ and $^{Endoscope}T_{LUS}$, a transformation between the ultrasound and CT image spaces can be computed:

$$^{CT}T_{LUS} = {}^{Endoscope}T_{CT}^{-1} * {}^{Endoscope}T_{LUS}.$$

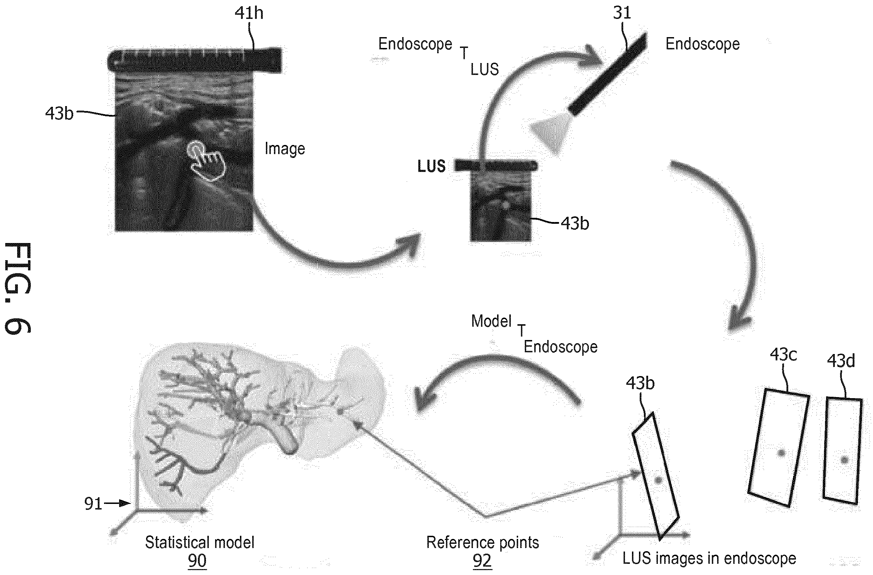

Anatomical Atlas Registration. The fusion of endoscopic/ultrasound images may be registered to an anatomical atlas of an anatomical organ that captures the shapes of the anatomical organ across the population. Such registration enables only a rough localization of LUS probe 41 in relation to the anatomical organ. For example, FIG. 6 illustrates a registration $^{MODEL}T_{ENDOSCOPE}$ between a model space 91 of an anatomical atlas 90 of a liver and endoscopy by using a set of pre-defined, quickly identifiable anatomical points of reference 92 shown in ultrasound images 43b-43d registered to the endoscopic image. These points of reference 92 may be organ specific large vessels bifurcations or surface landmarks.

To facilitate a further understanding of the present disclosure, the following description of FIGS. 5-16 teaches basic inventive principles of a display of an image fusion of an endoscopic image and an ultrasound image of the present disclosure with the endoscopic view or an anatomical model serving as the base image. From this description, those having ordinary skill in the art will appreciate how to apply the inventive principles for numerous additional embodiments of the present disclosure for displaying an image fusion of an endoscopic image and an ultrasound image.

Ultrasound Image Depth. In this display embodiment, a specific 2D planar ultrasound image of a 3D scan ultrasound image can be displayed to operator's preferences. More particularly, an operator can decide to overlay a specific 2D planar ultrasound image of a 3D scan ultrasound image on an endoscopic view or an anatomical model display serving as the base image.

FIGS. 7A and 7B illustrate an example whereby the operator can manipulate a graphical slider 64 to select between specific depths 43b and 43c of an 3D scan ultrasound image of a liver fused with a base image 100 of the liver (e.g., an endoscopic view or anatomical model view display).

Ultrasound Image/Instrument View. In this display embodiment, a previously recorded and saved 2D planar ultrasound image can be displayed under an instrument during the procedure and after an ultrasound scanning that generates a 3D scan ultrasound image. The 2D planar ultrasound image can further be transformed to put the 2D planar ultrasound image in perspective of the instrument.

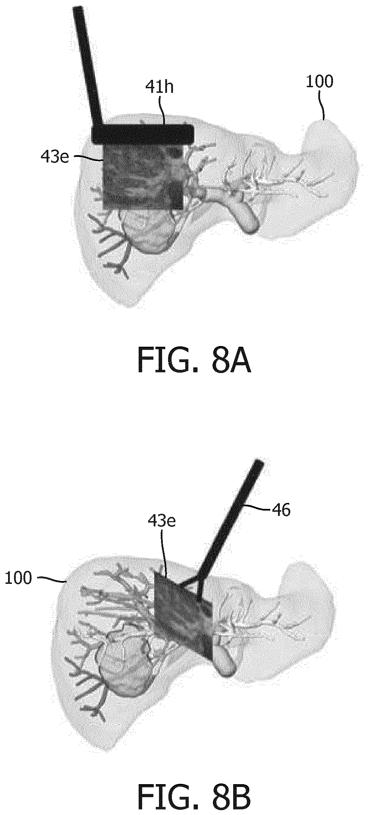

For example, FIGS. 8A and 8B illustrate an example whereby a previously recorded and saved 2D planar ultrasound image 43e of a targeted area of a liver is transformed and displayed under an instrument 46 during the procedure and after the scanning as an overlay on base image 100 (e.g., an endoscopic view, segmented volume view or an anatomical atlas view).

Ultrasound Image Perspective Transformation. In this display embodiment, either during the scanning or during the procedure, a base image (e.g., an endoscopic view, segmented volume view or an anatomical atlas view) can be recomputed and transformed using perspective so that the ultrasound image is shown in an undistorted perspective. This can be achieved using inverse perspective transformation as well known in art of the present disclosure.

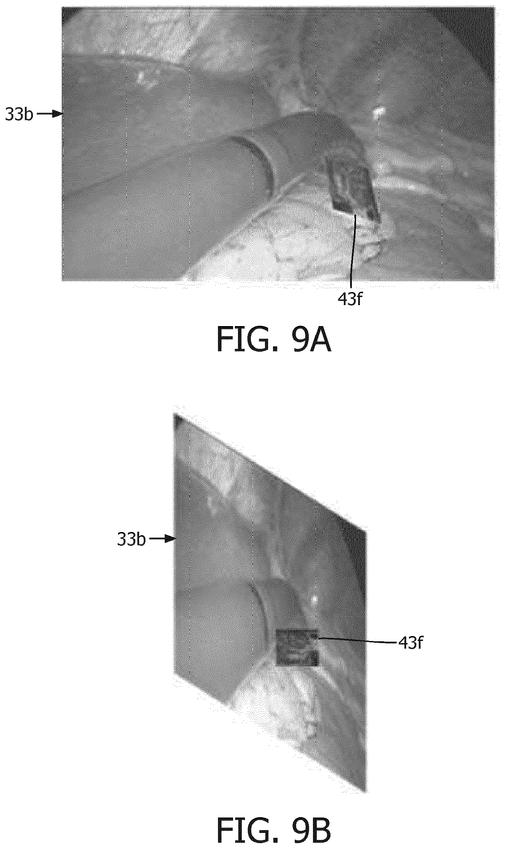

For example, FIGS. 9A and 9B illustrate an endoscope image 33b as recomputed and transformed to show an ultrasound image 43e in an undistorted perspective.

Model Reference in Endoscope View. In this display embodiment, using the aforementioned anatomical model transformations, each scanned 2D planar ultrasound image (or each 2D planar ultrasound image selected by the operator) may be displayed in context of the anatomical model (e.g., a preoperative CT model or an anatomical atlas). Additional reference planes selected in the anatomical model can be displayed to facilitate better use and selection of the 2D planar ultrasound images. The 2D planar ultrasound image can be visualized in endoscope view, anatomical model view or both.

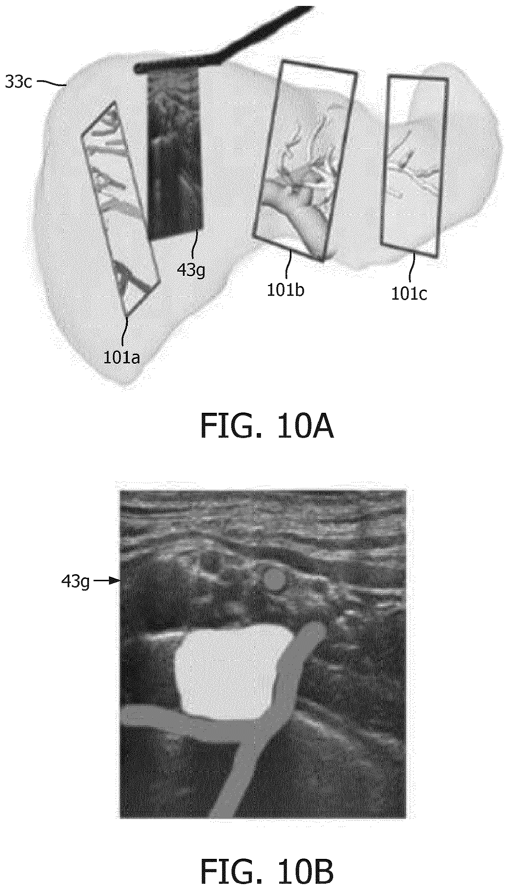

For example, FIG. 10A illustrates reference planes 101a-101c of preoperative CT information overlaid in conjunction with ultrasound image 43g on an endoscopic image 33c, and FIG. 10B illustrates ultrasound image 43g with preoperative CT information overlaid on ultrasound image 43g.

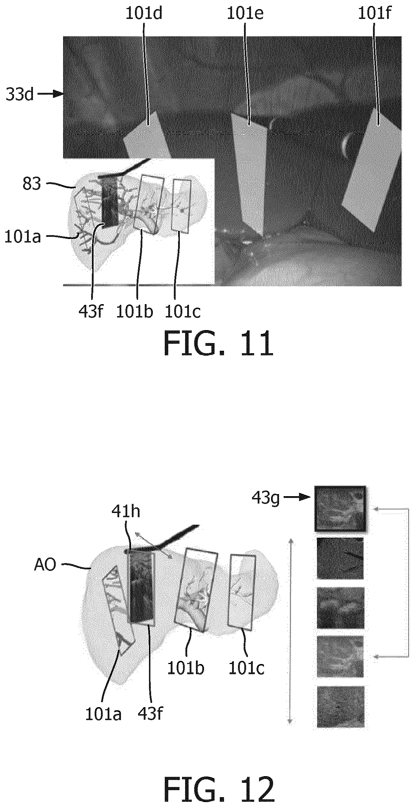

By further example, FIG. 11 illustrates reference planes 101d-101f in an endoscopic image 33d corresponding to reference planes 101a-101c of a CT image 83.

LUS Scanning. A technique of the present disclosure facilitates revisiting of same/similar planes during one scanning run or during revisiting of the scanning. The operator can use the saved LUS scanned positions in endoscope view to place the ultrasound images. Finding the right plane of imaging can be challenging due to 2D nature of endoscope images. In this display embodiment as exemplary shown in FIG. 12, the operator roughly positions LUS probe head 41h in the position close to the desired imaging plane (2D problem). After the placement, the operator pivots LUS probe head 41 as shown with the arrow and imaging fusion controller 50 collects a sequence 43g of ultrasound images. Using similarity metrics well known in art of the present disclosure (e.g. mutual information), imaging fusion controller 50 finds the most similar ultrasound image to the desired image position and notifies the operator when to stop pivoting.

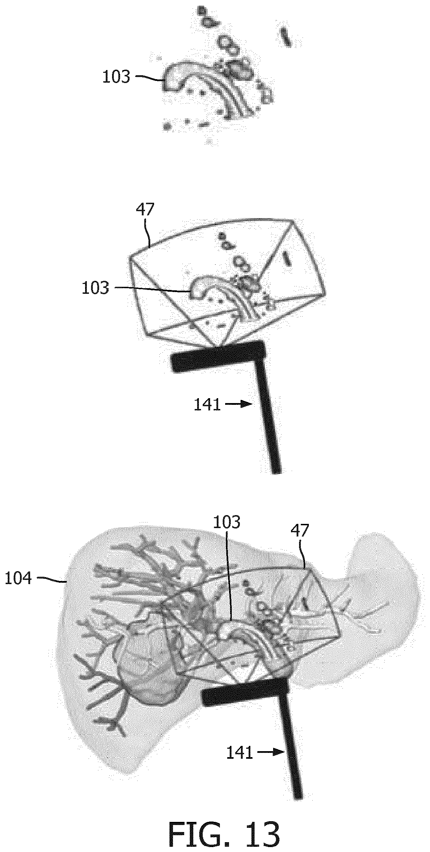

Virtual LUS Probe. In this embodiment, a virtual probe is displayed in conjunction with an anatomical model (e.g. 3D reconstruction of the organs surface and internal structures). In addition (or instead), an outline of ultrasound volume/plane can be displayed in conjunction with the anatomical model to facilitate better spatial orientation for the operator. In addition (or instead), the ultrasound image or volume can be overlaid over the anatomical model or 3D reconstruction. For example, FIG. 13 illustrates a 3D reconstruction of an anatomical structure 103 followed by a display of a virtual probe 141 that is then overlaid on an anatomical model 104 (e.g., CT image or an anatomical atlas).

Surgical Visualization. In this display embodiment, the process of the surgical procedure is tracked by visualizing the current and formerly visited/treated reference plane containing the tumor. First, an endoscope view is registered to an anatomical model (e.g., a preoperative 3D image or an anatomical atlas as previously described herein in connection with FIGS. 5 and 6). Second, based on this registration, an operator marks intraoperatively the tumor positions by acquiring and storing reference planes. Third, the system tracks the process of the procedure by visualizing the current and/or previously treated tumor. A current plane could be visualized as a blinking, red plane, whereas the plane containing the treated (ablated) tumor could be shown as black.

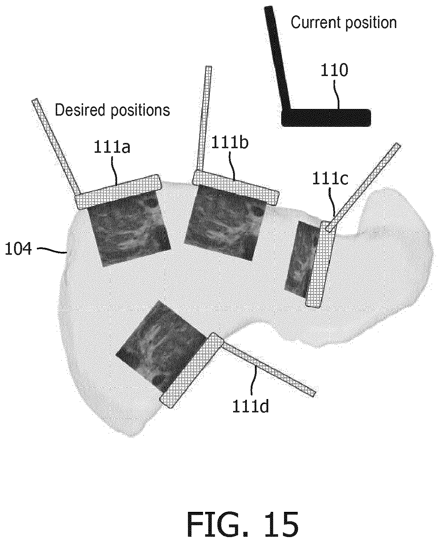

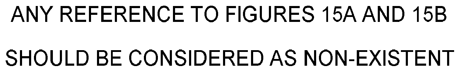

For example, FIGS. 15A and 15B show reference planes 101g-101i overlaid on base image 100 (e.g., an endoscopic view, segmented volume view or an anatomical atlas view) whereby FIG. 15A specifically show reference plane 101g as the current surgical reference plane and FIG. 15B specifically shows reference plane 101g representing a treated area and reference plane 101i is the current surgical reference plane.

Projected LUS Views. In order to visualize desired anatomical regions or lesions, a laparoscopic probe must be placed in the specific positions and orientations. This can be a challenging task in a minimally invasive setting due to mirrored motions and mismatch of coordinate systems between the LUS probe, the patient, and an anatomical model view). This display embodiment helps alleviate this difficulty by presenting an anatomical model of an anatomical organ (e.g., segmented CT image or anatomical atlas) along with an overlay of probe positions and orientations that would achieve the relevant views, such as, for example, a current position 110 and projected positions 111a-111d relative to anatomical mode image 104 (e.g., a segmented CT image or an anatomical atlas) shown in FIG. 15. These positions and orientations can be saved from views previously found manually, or computed from knowledge of locations of regions of interest. Additionally, if the laparoscopic probe is tracked, its current position and orientation can be overlaid with respect to the atlas to further help with probe placement.

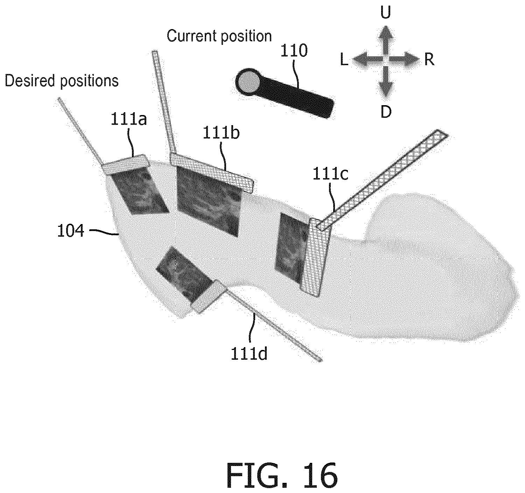

Transformed Projected LUS Views. Following the previous embodiment, the atlas viewpoint can be transformed into an intuitive perspective, such as a bird's eye view, user console point of view, or laparoscopic point of view of anatomical model image 104 (e.g., a segmented CT image or an anatomical atlas) as shown in FIG. 16 with current position 110 and projected positions 111a-111d. Such transformation allows the clinician to indicate probe movements in his/her own frame of reference, and these directions are transformed into the appropriate motions in the anatomical model coordinate system. Inversely, probe motions required to obtain desired views in the anatomical model coordinate system can be converted into motions in the clinician's frame of reference.

In practice, image fusion controller 50 (FIG. 1) may be installed within or linked to a workstation.

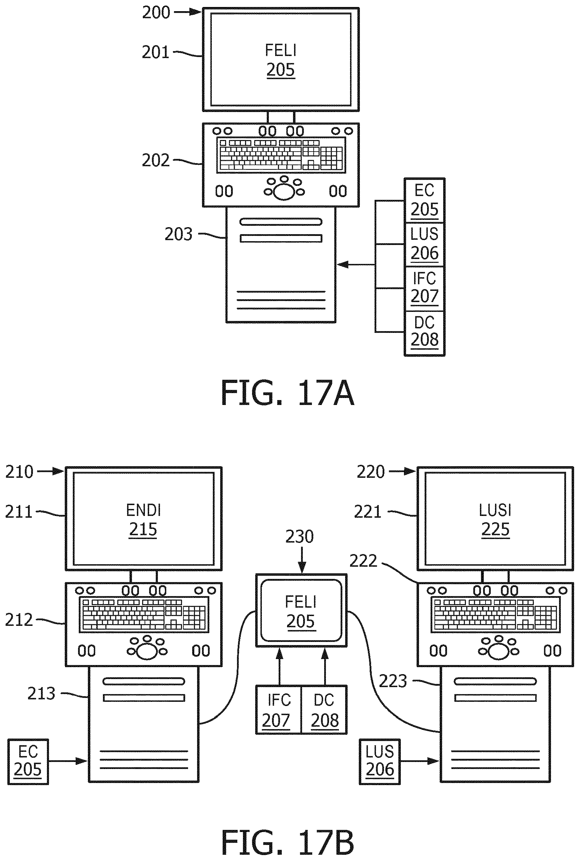

For example, FIG. 17A shows an image fusion workstation 200 employing a monitor 201, an interface platform 202 (e.g., keyboard, buttons, dials, joysticks, etc.), a client computer 203, and a controller network 204 installed within workstation computer 203.

Controller network 204 includes an endoscope controller 205, a LUS probe controller 206, an image fusion controller 207 and a display controller 208 in accordance with the inventive principles of the present disclosure. In practice, controllers 205-208 may be segregated and/or integrated to any degree within client computer 203. Alternatively, as shown in FIG. 17B, endoscope controller 205 may be installed within an endoscopy workstation 210 (having a monitor 211, an interface platform 212 and a client computer 213) and LUS probe controller 206 may be installed within n LUS workstation 220 (having a monitor 221, an interface platform 222 and a client computer 223) whereby image fusion controller 207 and display controller 208 are installed within a workstation tablet 230 linked to workstations 210 and 220.

Referring to FIGS. 1-17, those having ordinary skill in the art will appreciate numerous benefits of the present disclosure including, but not limited to, the image-based fusion of the present disclosure of an endoscopic image and an ultrasound image of the anatomical region as a significant improvement to existing systems, workstations, controllers and methods incorporated into minimally invasive procedures involving a tracking-based fusion of the endoscopic image and the ultrasound image of the anatomical region.

Furthermore, as one having ordinary skill in the art will appreciate in view of the teachings provided herein, features, elements, components, etc. described in the present disclosure/specification and/or depicted in the Figures may be implemented in various combinations of electronic components/circuitry, hardware, executable software and executable firmware and provide functions which may be combined in a single element or multiple elements. For example, the functions of the various features, elements, components, etc. shown/illustrated/depicted in the Figures can be provided through the use of dedicated hardware as well as hardware capable of executing software in association with appropriate software. When provided by a processor, the functions can be provided by a single dedicated processor, by a single shared processor, or by a plurality of individual processors, some of which can be shared and/or multiplexed. Moreover, explicit use of the term "processor" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, circuitry, combinations thereof, etc.) which is capable of (and/or configurable) to perform and/or control a process.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar function, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Furthermore, exemplary embodiments of the present disclosure can take the form of a computer program product or application module accessible from a computer-usable and/or computer-readable storage medium providing program code and/or instructions for use by or in connection with, e.g., a computer or any instruction execution system. In accordance with the present disclosure, a computer-usable or computer readable storage medium can be any apparatus that can, e.g., include, store, communicate, propagate or transport the program for use by or in connection with the instruction execution system, apparatus or device. Such exemplary medium can be, e.g., an electronic, magnetic, optical, electromagnetic, infrared or semiconductor system (or apparatus or device) or a propagation medium. Examples of a computer-readable medium include, e.g., a semiconductor or solid state memory, magnetic tape, a removable computer diskette, a random access memory (RAM), a read-only memory (ROM), flash (drive), a rigid magnetic disk and an optical disk. Current examples of optical disks include compact disk - read only memory (CD-ROM), compact disk - read/write (CD-R/W) and DVD. Further, it should be understood that any new computer-readable medium which may hereafter be developed should also be considered as computer-readable medium as may be used or referred to in accordance with exemplary embodiments of the present disclosure and disclosure.

Having described preferred and exemplary embodiments of novel and inventive image-based fusion of an endoscopic image and a ultrasound image of the anatomical region, (which embodiments are intended to be illustrative and not limiting), it is noted that modifications and variations can be made by persons having ordinary skill in the art in light of the teachings provided herein, including the Figures. It is therefore to be understood that changes can be made in/to the preferred and exemplary embodiments of the present disclosure which are within the scope of the embodiments disclosed herein.

Moreover, it is contemplated that corresponding and/or related systems incorporating and/or implementing the device or such as may be used/implemented in a device in accordance with the present disclosure are also contemplated and considered to be within the scope of the present disclosure. Further, corresponding and/or related method for manufacturing and/or using a device and/or system in accordance with the present disclosure are also contemplated and considered to be within the scope of the present disclosure.

**Claims**

1. An image fusion workstation for an image-based fusion of an endoscopic image (33) of an anatomical region generated by an endoscope (31) and an ultrasound image (43) of the anatomical region generated by a laparoscopic ultrasound probe (41), the image fusion workstation comprising:

   an image fusion controller (50),
   wherein, responsive to a communication of the endoscopic image (33) and the ultrasound image (43), the image fusion controller (50) is configured to control the fusion between the endoscopic image (33) and the ultrasound image (43) based on an image transformation between an endoscopic image space of the endoscope (31) and an ultrasound image space of the laparoscopic ultrasound probe (41) derived from a detection by the image fusion controller (50) of the laparoscopic ultrasound probe (41) within a field-of-view (32) of the endoscope (31) of the anatomical region; and
   a display controller (60),
   wherein the display controller (60) is configured to control a display of the fusion by the image fusion controller (50) of the endoscopic image (33) and the ultrasound image (43).

2. The image fusion workstation of claim 1,
   wherein the image fusion controller (50) and the display controller (60) are further configured to control a user interactive selection of a depth of the ultrasound image (43) from the laparoscopic ultrasound probe (41).

3. The image fusion workstation of claim 1,
   wherein the image fusion controller (50) and the display controller (60) are further configured to control a display of the ultrasound image (43) relative to an instrument inserted into the anatomical region.

4. The image fusion workstation of claim 1,
   wherein the image fusion controller (50) and the display controller (60) are further configured to control a perspective view of the display of the ultrasound image (43).

5. The image fusion workstation of claim 1,
   wherein, responsive to a communication of an anatomical model of the anatomical region, the image fusion controller (50) is configured to control a registration between the anatomical model of the anatomical region and the fusion of the endoscopic image and the ultrasound image based on an image transformation between a volumetric image space of the anatomical model and the endoscopic image space of the endoscope (31).

6. The image fusion workstation of claim 5, wherein the anatomical model is an anatomical atlas of an anatomical structure within the anatomical region.

7. The image fusion workstation of claim 5,
   wherein the image fusion controller (50) and the display controller (60) are further configured to control a display of at least one reference planar view of the anatomical model overlaid on a display of at least one of the endoscopic image (33), the ultrasound image (43) and the fusion of the endoscopic image (33) and the ultrasound image (43).

8. The image fusion workstation of claim 5,
   wherein the image fusion controller (50) and the display controller (60) are further configured to control a display of a targeted ultrasound image among a plurality of ultrasound images of the anatomical region generated by the laparoscope ultrasound probe (41) based on the registration between the anatomical model of the anatomical region and the fusion of the endoscopic image and the ultrasound image.

9. The image fusion workstation of claim 5,
   wherein the image fusion controller (50) and the display controller (60) are further configured to control a display of a virtual laparoscopic probe and the ultrasound image in conjunction with a display of the anatomical model based on the registration between the anatomical model of the anatomical region and the fusion of the endoscopic image and the ultrasound image.

10. The image fusion workstation of claim 1,
    wherein the image fusion controller (50) and the display controller (60) further control a display of the ultrasound image indicative of a status of a surgical procedure performed within the anatomical region.

11. The image fusion workstation of claim 5,
    wherein the image fusion controller (50) and the display controller (60) are further configured to control a display of at least one reference planar view of the anatomical model indicative of a status of a surgical procedure performed within the anatomical region.

12. The image fusion workstation of claim 5,
    wherein the image fusion controller (50) and the display controller (60) are further configured to control a virtual display of at least one desired position of the laparoscopic ultrasound probe (41) relative to the anatomical model based on the registration between the anatomical model of the anatomical region and the fusion of the endoscopic image and the ultrasound image.

13. The image fusion workstation of claim 1 wherein the image fusion controller (50) further comprises:

   a probe detector (51) configured to control a detection of the laparoscopic ultrasound probe (41) within a field-of-view of the endoscope (31);

an image transformer (52) configured to control a computation of an image transformation between the endoscopic image space of the endoscope (31) and the ultrasound image space of the laparoscopic ultrasound probe (41) derived from the detection by the probe detector (51) of the laparoscopic ultrasound probe (41) within the field-of-view of the endoscope (31); and

an image integrator (53) configured to control the fusion of the endoscopic image (33) and the ultrasound image (43) based on the image transformation computed by the image transformer (52).

14. A method for an image-based fusion of an endoscopic image (33) of an anatomical region generated by an endoscope (31) and an ultrasound image (43) of the anatomical region generated by a laparoscopic ultrasound probe (41), the method comprising:

detecting the laparoscopic ultrasound probe (41) within a field-of-view (32) of the endoscope (31) of the anatomical region;

computing an image transformation between an endoscopic image space of the endoscope (31) and an ultrasound image space of the laparoscopic ultrasound probe (41) by deriving the image transformation from the detection of the laparoscopic ultrasound probe (41) within a field-of-view (32) of the endoscope (31) of the anatomical region; and

fusing the endoscopic image (33) and the ultrasound image (43) based on the image transformation.

15. Computer program product comprising instructions which when executed on a computer cause the computer to carry out the method according to claim 14.

**Patentansprüche**

1. Bildfusions-Workstation für eine bildbasierte Fusion eines endoskopischen Bildes (33) einer anatomischen Region, das von einem Endoskop (31) erzeugt wird, und eines Ultraschallbilds (43) der anatomischen Region, das von einer laparoskopischen Ultraschallsonde (41) erzeugt wird, wobei die Bildfusions-Workstation umfasst:

einen Bildfusions-Controller (50),

wobei der Bildfusions-Controller (50) dafür ausgelegt ist, als Reaktion auf eine Übermittlung des endoskopischen Bildes (33) und des Ultraschallbilds (43) die Fusion zwischen dem endoskopischen Bild (33) und dem Ultraschallbild (43) zu steuern, auf Basis einer Bildtransformation zwischen einem Endoskopiebildraum des Endoskops (31) und einem Ultraschallbildraum der laparoskopischen Ultraschallsonde (41), die aus einer Erfassung der laparoskopischen Ultraschallsonde (41) durch den Bildfusions-Controller (50) innerhalb eines Sichtfelds (32) des Endoskops (31) in der anatomischen Region abgeleitet wird; und

einen Anzeigen-Controller (60),

wobei der Anzeigen-Controller (60) dafür ausgelegt ist, eine Anzeige der Fusion des Bildfusions-Controllers (50) aus dem endoskopischen Bild (33) und dem Ultraschallbild (43) zu steuern.

2. Bildfusions-Workstation nach Anspruch 1,

wobei der Bildfusions-Controller (50) und der Anzeige-Controller (60) ferner dafür ausgelegt sind, eine Benutzerinteraktionsauswahl einer Tiefe des Ultraschallbilds (43) ab der laparoskopischen Ultraschallsonde (41) zu steuern.

3. Bildfusions-Workstation nach Anspruch 1,

wobei der Bildfusions-Controller (50) und der Anzeige-Controller (60) ferner dafür ausgelegt sind, eine Anzeige des Ultraschallbilds (43) in Bezug auf ein Instrument, das in die anatomische Region eingeführt wird, zu steuern.

4. Bildfusions-Workstation nach Anspruch 1,

wobei der Bildfusions-Controller (50) und der Anzeige-Controller (60) ferner dafür ausgelegt sind, eine perspektivische Darstellung der Anzeige des Ultraschallbilds (43) zu steuern.

5. Bildfusions-Workstation nach Anspruch 1,

wobei der Bildfusions-Controller (50) dafür ausgelegt ist, als Reaktion auf eine Übermittlung eines anatomischen Modells der anatomischen Region eine Registrierung zwischen dem anatomischen Modell der anatomischen Region und der Fusion des endoskopischen Bildes und des Ultraschallbilds auf Basis einer Bildtransformation zwischen einem volumetrischen Bildraum des anatomischen Modells und dem endoskopischen Bildraum des Endoskops (31) zu steuern.

6. Bildfusions-Workstation nach Anspruch 5, wobei das anatomische Modell ein anatomischer Atlas einer anatomischen Struktur innerhalb der anatomischen Region ist.

7. Bildfusions-Workstation nach Anspruch 5,
wobei der Bildfusions-Controller (50) und der Anzeige-Controller (60) ferner dafür ausgelegt sind, eine Anzeige von mindestens einer Bezugs-Planansicht des anatomischen Modells, die über eine Anzeige von mindestens einem von dem endoskopischen Bild (33), dem Ultraschallbild (43) und der Fusion aus dem endoskopischen Bild (33) und dem Ultraschallbild (43) gelegt ist, zu steuern.

8. Bildfusions-Workstation nach Anspruch 5,
wobei der Bildfusions-Controller (50) und der Anzeige-Controller (60) ferner dafür ausgelegt sind, eine Anzeige eines zum Ziel genommenen Ultraschallbilds von einer Vielzahl von Ultraschallbildern der anatomischen Region, die durch die laparoskopische Ultraschallsonde (41) erzeugt worden sind, auf Basis der Registrierung zwischen dem anatomischen Modell der anatomischen Region und der Fusion des endoskopischen Bildes und des Ultraschallbilds zu steuern.

9. Bildfusions-Workstation nach Anspruch 5,
wobei der Bildfusions-Controller (50) und der Anzeige-Controller (60) ferner dafür ausgelegt sind, eine Anzeige einer virtuellen laparoskopischen Sonde und des Ultraschallbilds in Verbindung mit einer Anzeige des anatomischen Modells auf Basis der Registrierung zwischen dem anatomischen Modell der anatomischen Region und der Fusion des endoskopischen Bildes und des Ultraschallbilds zu steuern.

10. Bildfusions-Workstation nach Anspruch 1,
wobei der Bildfusions-Controller (50) und der Anzeige-Controller (60) ferner eine Anzeige des Ultraschallbilds steuern, die einen Status eines chirurgischen Eingriffs angibt, der innerhalb der anatomischen Region durchgeführt wird.

11. Bildfusions-Workstation nach Anspruch 5,
wobei der Bildfusions-Controller (50) und der Anzeige-Controller (60) ferner dafür ausgelegt sind, eine Anzeige von mindestens einer Bezugs-Planansicht des anatomischen Modells zu steuern, die einen Status eines chirurgischen Eingriffs angibt, der innerhalb der anatomischen Region durchgeführt wird.

12. Bildfusions-Workstation nach Anspruch 5,
wobei der Bildfusions-Controller (50) und der Anzeige-Controller (60) ferner dafür ausgelegt sind, eine virtuelle Anzeige mindestens einer gewünschten Position der laparoskopischen Ultraschallsonde (41) in Bezug auf das anatomische Modell auf Basis der Registrierung zwischen dem anatomischen Modell der anatomischen Region und der Fusion des endoskopischen Bildes und des Ultraschallbilds zu steuern.

13. Bildfusions-Workstation nach Anspruch 1, wobei der Bildfusions-Controller (50) ferner umfasst:

einen Sondendetektor (51), der dafür ausgelegt ist,
eine Erfassung der laparoskopischen Ultraschallsonde (41) innerhalb eines Sichtfelds des Endoskops (31) zu steuern;
einen Bildtransformator (52), der dafür ausgelegt ist, eine Berechnung einer Bildtransformation zwischen dem endoskopischen Bildraum des Endoskops (31) und dem Ultraschallbildraum der laparoskopischen Ultraschallsonde (41) zu steuern, die aus der Erfassung der laparoskopischen Ultraschallsonde (41) innerhalb des Sichtfelds des Endoskops (31) durch den Sondendetektor (51) abgeleitet ist; und
einen Bildintegrator (53), der dafür ausgelegt ist, die Fusion des endoskopischen Bildes (33) und des Ultraschallbilds (43) auf Basis der durch den Bildtransformator (52) berechneten Bildtransformation zu steuern.

14. Verfahren für eine bildbasierte Fusion eines endoskopischen Bildes (33) einer anatomischen Region, das von einem Endoskop (31) erzeugt wird, und eines Ultraschallbildes (43) der anatomischen Region, das von einer laparoskopischen Ultraschallsonde (41) erzeugt wird, wobei das Verfahren umfasst:

Erfassen der laparoskopischen Ultraschallsonde (41) innerhalb eines Sichtfelds (32) des Endoskops (31) in der anatomischen Region;
Berechnen einer Bildtransformation zwischen einem endoskopischen Bildraum des Endoskops (31) und einem Ultraschallbildraum der laparoskopischen Ultraschallsonde (41) durch Ableiten der Bildtransformation aus der Erfassung der laparoskopischen Ultraschallsonde (41) innerhalb des Sichtfelds (32) des Endoskops (31) der anatomischen Region; und

Fusionieren des endoskopischen Bildes (33) und des Ultraschallbilds (43) auf Basis der Bildtransformation.

15. Computerprogrammprodukt, das Befehle umfasst, die bei ihrer Ausführung auf einem Computer den Computer veranlassen, das Verfahren nach Anspruch 14 auszuführen.

## Revendications

1. Poste de travail destiné à la fusion d'images pour une fusion basée sur l'image d'une image endoscopique (33) d'une zone anatomique générée par un endoscope (31) et d'une image ultrasonore (43) de la zone anatomique générée par une sonde à ultrasons laparoscopique (41), ledit poste de travail destiné à la fusion d'images comprenant :

   un dispositif de commande de fusion d'image (50),
   dans lequel, en réponse à une communication de l'image endoscopique (33) et de l'image ultrasonore (43), le dispositif de commande de fusion d'image (50) est conçu pour commander la fusion entre l'image endoscopique (33) et l'image ultrasonore (43) en fonction d'une transformation d'image entre un espace d'image endoscopique de l'endoscope (31) et un espace d'image ultrasonore de la sonde à ultrasons laparoscopique (41) dérivée d'une détection par le dispositif de commande de fusion d'image (50) de la sonde à ultrasons laparoscopique (41) dans un champ de vision (32) de l'endoscope (31) de la zone anatomique ; et un dispositif de commande d'affichage (60),
   dans lequel le dispositif de commande d'affichage (60) est conçu pour commander un affichage de la fusion par le dispositif de commande de fusion d'image (50) de l'image endoscopique (33) et de l'image ultrasonore (43).

2. Poste de travail destiné à la fusion d'images selon la revendication 1, dans lequel le dispositif de commande de fusion d'image (50) et le dispositif de commande d'affichage (60) sont en outre conçus pour commander une sélection interactive utilisateur d'une profondeur de l'image ultrasonore (43) à partir de la sonde à ultrasons laparoscopique (41).

3. Poste de travail destiné à la fusion d'images selon la revendication 1, dans lequel le dispositif de commande de fusion d'image (50) et le dispositif de commande d'affichage (60) sont en outre conçus pour commander un affichage de l'image ultrasonore (43) par rapport à un instrument inséré dans la zone anatomique.

4. Poste de travail destiné à la fusion d'images selon la revendication 1, dans lequel le dispositif de commande de fusion d'image (50) et le dispositif de commande d'affichage (60) sont en outre conçus pour commander une vue en perspective de l'affichage de l'image ultrasonore (43).

5. Poste de travail destiné à la fusion d'images selon la revendication 1, dans lequel, en réponse à une communication d'un modèle anatomique de la zone anatomique, le dispositif de commande de fusion d'image (50) est conçu pour commander un alignement entre le modèle anatomique de la zone anatomique et la fusion de l'image endoscopique et de l'image ultrasonore en fonction d'une transformation d'image entre un espace d'image volumétrique du modèle anatomique et l'espace d'image endoscopique de l'endoscope (31).

6. Poste de travail destiné à la fusion d'images selon la revendication 5, dans lequel le modèle anatomique est un atlas anatomique d'une structure anatomique dans la zone anatomique.

7. Poste de travail destiné à la fusion d'images selon la revendication 5, dans lequel le dispositif de commande de fusion d'image (50) et le dispositif de commande d'affichage (60) sont en outre conçus pour commander un affichage d'au moins une vue plane de référence du modèle anatomique superposé sur un affichage de l'image endoscopique (33) et/ou de l'image ultrasonore (43) et/ou de la fusion de l'image endoscopique (33) et de l'image ultrasonore (43).

8. Poste de travail destiné à la fusion d'images selon la revendication 5, dans lequel le dispositif de commande de fusion d'image (50) et le dispositif de commande d'affichage (60) sont en outre conçus pour commander un affichage d'une image ultrasonore ciblée parmi une pluralité d'images ultrasonores de la zone anatomique générées par la sonde à ultrasons laparoscopique (41) en fonction de l'enregistrement entre le modèle anatomique de la zone anatomique et la fusion de l'image endoscopique et de l'image ultrasonore.

9. Poste de travail destiné à la fusion d'images selon la revendication 5, dans lequel le dispositif de commande de fusion d'image (50) et le dispositif de commande d'affichage (60) sont en outre conçus pour commander un affichage d'une sonde laparoscopique virtuelle et de l'image ultrasonore conjointement avec un affichage du modèle anatomique en fonction de l'enregistrement entre le modèle anatomique de la zone anatomique et la fusion de l'image endoscopique et de l'image ultrasonore.

10. Poste de travail destiné à la fusion d'images selon la revendication 1, dans lequel le dispositif de commande de fusion d'image (50) et le dispositif de commande d'affichage (60) commandent en outre un affichage de l'image ultrasonore indicative d'un état d'une intervention chirurgicale réalisée dans la zone anatomique.

11. Poste de travail destiné à la fusion d'images selon la revendication 5, dans lequel le dispositif de commande de fusion d'image (50) et le dispositif de commande d'affichage (60) sont en outre conçus pour commander un affichage d'au moins une vue plane de référence du modèle anatomique indicative d'un état d'une intervention chirurgicale réalisée dans la zone anatomique.

12. Poste de travail destiné à la fusion d'images selon la revendication 5, dans lequel le dispositif de commande de fusion d'image (50) et le dispositif de commande d'affichage (60) sont en outre conçus pour commander un affichage virtuel d'au moins une position souhaitée de la sonde à ultrasons laparoscopique (41) par rapport au modèle anatomique en fonction de l'enregistrement entre le modèle anatomique de la zone anatomique et la fusion de l'image endoscopique et de l'image ultrasonore.

13. Poste de travail destiné à la fusion d'images selon la revendication 1, dans lequel le dispositif de commande de fusion d'image (50) comprend en outre :

un détecteur de sonde (51) conçu pour commander une détection de la sonde à ultrasons laparoscopique (41) dans un champ de vision de l'endoscope (31) ;
un transformateur d'image (52) conçu pour commander un calcul d'une transformation d'image entre l'espace d'image endoscopique de l'endoscope (31) et l'espace d'image ultrasonore de la sonde à ultrasons laparoscopique (41) dérivée de la détection par le détecteur de sonde (51) de la sonde à ultrasons laparoscopique (41) dans le champ de vision de l'endoscope (31) ; et un intégrateur d'image (53) conçu pour commander la fusion de l'image endoscopique (33) et de l'image ultrasonore (43) en fonction de la transformation d'image calculée par le transformateur d'image (52).

14. Procédé pour une fusion à base d'image d'une image endoscopique (33) d'une zone anatomique générée par un endoscope (31) et d'une image ultrasonore (43) de la zone anatomique générée par une sonde à ultrasons laparoscopique (41), ledit procédé comprenant :

la détection de la sonde à ultrasons laparoscopique (41) dans un champ de vision (32) de l'endoscope (31) de la zone anatomique ;
le calcul d'une transformation d'image entre un espace d'image endoscopique de l'endoscope (31) et un espace d'image ultrasonore de la sonde à ultrasons laparoscopique (41) par dérivation de la transformation d'image à partir de la détection de la sonde à ultrasons laparoscopique (41) dans un champ de vue (32) de l'endoscope (31) de la zone anatomique ; et la fusion de l'image endoscopique (33) et de l'image ultrasonore (43) en fonction de la transformation d'image.

15. Produit de programme informatique comprenant des instructions, lesquelles, lorsqu'elles sont exécutées sur un ordinateur, amènent l'ordinateur à mettre en œuvre le procédé selon la revendication 14.

| Endscope imaging Phase 11 Field-of-view | Lus imaging phase 12 2d planar us image 3d scan us image | Imagine fusion Phase 13 Endoscope ←→ plus |
|---|---|---|

10 →

| Endscope Controller 30 | Lus probe Controller 40 | Imagine fusion Controller 50 | Display Controller 60 |
|---|---|---|---|

20 →

# FIG. 1

| Endoscope controller 30 | | | Lus probe controller 40 |

AR

31

32   41h

42

41

EIF 33

USI 43

| Image fusion controller 50 |
|---|
| Probe detector 51 | Image transformer 52 | Image integrator 53 |

| Display / monitor 62 | Feui 62 | Display controller 60 | lfd 54 |
| | | | lic 63 |

# FIG. 2

FIG. 3

$$\begin{bmatrix} u \\ v \\ 1 \end{bmatrix} = KP \begin{bmatrix} x \\ y \\ z \\ 1 \end{bmatrix}$$

FIG. 4A

$$\begin{bmatrix} u \\ v \\ 1 \end{bmatrix} = KP \begin{bmatrix} x \\ y \\ z \\ 1 \end{bmatrix}$$

$$\begin{bmatrix} u \\ v \\ 1 \end{bmatrix} = KP \begin{bmatrix} x \\ y \\ z \\ 1 \end{bmatrix}$$

FIG. 4B

ENDOSCOPE
34

CT
81

FIG. 5
(Prior art)

FIG. 6

FIG. 7A

FIG. 7B

FIG. 8A

FIG. 8B

FIG. 9A

FIG. 9B

33c

43g

101a

101b

101c

## FIG. 10A

43g

## FIG. 10B

FIG. 11

FIG. 12

FIG. 13

FIG. 14A

FIG. 14B

FIG. 15

ANY REFERENCE TO FIGURES 15A AND 15B

SHOULD BE CONSIDERED AS NON-EXISTENT

FIG. 16

200 →

201 —

FELI
205

202 —

203 —

EC
205

LUS
206

IFC
207

DC
208

# FIG. 17A

210 →

211 —

ENDI
215

212 —

213 —

EC
205

230

222 —

FELI
205

IFC
207

DC
208

220 →

221 —

LUSI
225

223 —

LUS
206

# FIG. 17B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015091226 A

- US 9095252 B2